(19)

Europäisches
Patentamt

European
Patent Office

Office européen
des brevets

(11) **EP 2 337 866 B1**

## (12) EUROPEAN PATENT SPECIFICATION

(45) Date of publication and mention
of the grant of the patent:
**30.07.2014 Bulletin 2014/31**

(51) Int Cl.:
**G06F 19/24** *(2011.01)*  **G01N 33/68** *(2006.01)*

(21) Application number: **09821274.9**

(22) Date of filing: **15.10.2009**

(86) International application number:
**PCT/US2009/060895**

(87) International publication number:
**WO 2010/045490 (22.04.2010 Gazette 2010/16)**

(54) **HUMAN BIOMARKER HYPERMAPPING FOR DEPRESSIVE DISORDERS**

HYPERKARTIERUNG MENSCHLICHER BIOMARKER FÜR DEPRESSIVE STÖRUNGEN

MAPPAGE SUR HYPERESPACE MULTIDIMENSIONNEL DE BIOMARQUEUR HUMAIN POUR DES TROUBLES DÉPRESSIFS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **15.10.2008 US 105641 P**

(43) Date of publication of application:
**29.06.2011 Bulletin 2011/26**

(73) Proprietor: **Ridge Diagnostics, Inc.**
**La Jolla, California 92037 (US)**

(72) Inventors:
• **PI, Bo**
**Carlsbad, CA 92009 (US)**
• **BILELLO, John**
**Durham, NC 27713 (US)**

(74) Representative: **Grund, Martin**
**Grund**
**Intellectual Property Group**
**Postfach 44 05 16**
**80754 München (DE)**

(56) References cited:
**WO-A2-2009/111595    WO-A2-2011/094308**

• **LOPEZ-LEON ET AL.:** 'Meta-analyses of genetic studies on major depressive disorder' MOL. PSYCHIATRY vol. 13, no. 8, 16 October 2007, pages 772 - 785, XP008146289
• **BERTHOLD-LOSLEBEN ET AL.:** 'The TNF-alpha system: functional aspects in depression, narcolepsy and psychopharmacology' CURR. NEUROPHARMACOL. vol. 6, no. 3, September 2002, pages 193 - 202, XP008146423
• **PARIANTE ET AL.:** 'The HPA axis in major depression: classical theories and new developments' TRENDS NEROSCI. vol. 31, no. 9, 31 July 2008, pages 464 - 468, XP024524194
• **HUANG ET AL.:** 'Insulin sensitivity, proinflammatory markers and adiponectin in young males with different subtypes of depressive disorder' CLIN. ENDOCRINOL. vol. 67, no. 5, 13 August 2007, pages 784 - 789, XP008146291
• **SCHROETER ET AL.:** 'Serum markers support disease-specific glial pathology in major depression' J. AFFECT. DISORD. vol. 111, no. 2-3, 21 April 2008, pages 271 - 280, XP025571589

EP 2 337 866 B1

Description

TECHNICAL FIELD

This document relates to in vitro methods for assessing the probability that an individual has major depressive disorder (MDD) using algorithms and hypermapping based on a combination of parameters.

BACKGROUND

People can live with neuropsychiatric conditions for extended lengths of time In fact, neuropsychiatric conditions result in more years lived with disability (YLDs) than any other type of condition, accounting for almost 30 percent of total YLDs (Murray and Lopez (1996) Global Health Statistics: A Compendium of Incidence, Prevalence and Mortality Estimates for over 2000 Conditions Cambridge: Harvard School of Public Health). Several factors may contribute to sustained disability and less than optimal treatment outcomes, including inaccurate diagnosis, early discontinuation of treatment by clinicians, social stigma, inadequate antidepressant dosing, antidepressant side effects, and non-adherence to treatment by patients.

Most clinical disorders, including neuropsychiatric conditions such as depression disorder conditions (e.g., major depressive disorder (MDD)), do not arise due to a single biological change, but rather result from an interaction of multiple factors. Thus, different individuals affected by the same clinical condition (e.g., MDD) may present with different types or ranges of symptoms, depending on the specific changes within each individual. There is a need, however, for reliable methods for diagnosing or determining predisposition to MDD, as well as for assessing disease status and response to treatment on an individual basis.

López-León et al. (Molecular Psychiatry 2007, 13(8):772-785) describe a method for meta-analyzing several studies on major depressive disorders. Other studies (Berthold-Losleben and Himmerich, Current Neuropharmacology 2002, 6(3):193-202; Pariante and Lightman, Trends in Neurosciences 2008, 31(9):464-468; Hung et al., Clinical Endocrinology 2007, 67(5):784-789; Schroeter et al., Journal of Affective Disorders 2008, 111(2-3):271-280) address, inter alia, the correlation of several types of biological markers, hypothalamo-pituitary-adrenocortical axis and major depression.

SUMMARY

Traditional approaches to biomarkers often have included analyzing single markers or groups of single markers. Other approaches have included using algorithms to derive a single value that reflects disease status, prognosis, and/or response to treatment. Highly multiplexed microarray-based immunological tools can be used to simultaneously measure a plurality of parameters. An advantage of using such tools is that all results can be derived from the same sample and run under the same conditions at the same time. High-level pattern recognition approaches can be applied, and a number of tools are available, including clustering approaches such as hierarchical clustering, self-organizing maps, and supervised classification algorithms (e.g., support vector machines, k-nearest neighbors, hypermapping and neural networks). The latter group of analytical approaches is likely to be of substantial clinical use.

This document is based in part on the identification of methods for using hypermapping to determine diagnosis, prognosis, or predisposition to depression disorder conditions, and also to determine response to therapy. In addition, this document is based on the identification of methods for using hypermapping to determine diagnosis, prognosis, or predisposition to conditions such as infectious or chronic diseases. The methods can include, for example, selecting groups of biomarkers that may be related to a particular condition, obtaining clinical data from subjects for the selected groups of biomarkers, applying an optimization algorithm to the clinical data in order to arrive at coefficients for selected biomarkers within each group, creating a hypermap by developing vectors for each group of biomarkers, and using the hypermap to generate a diagnosis or decision (e.g., related to treatment or disease status) for an individual who may or may not have the condition. In some embodiments, for example, algorithms and hypermaps incorporating data from multiple biomarkers in biological samples such as serum or plasma can be developed for patient stratification, identification of pharmacodynamic markers, and monitoring treatment outcome.

The invention can be defined by the appendent claims.

In one aspect, an *in vitro* method for assessing the probability that an individual has major depressive disorder (MDD) is provided, comprising (a) measuring the levels of two or more groups of biomarkers in one or more serum, plasma, or blood samples from said subject, wherein said two or more groups of biomarkers are relevant to MDD and are selected from the group consisting of inflammatory biomarkers, hypothalamic-pituitary-adrenal (HPA) axis biomarkers, metabolic biomarkers, and neurotrophic biomarkers, wherein each group comprises two or more biomarkers; and wherein said inflammatory biomarkers comprise one or more of alpha 1 antitrypsin, alpha 2 macroglobulin, apolipoprotein CIII, CD40 ligand, interleukin 6, interleukin 13, interleukin 18, interleukin 1 receptor antagonist, myeloperoxidase, plasminogen activator inhibitor-1, RANTES (CCL5), soluble tumor necrosis factor alpha receptor II, and tumor necrosis factor alpha; said HPA axis biomarkers comprise one or more of cortisol, epidermal growth factor, granulocyte colony stimulating factor, pancreatic polypeptide, adrenocorticotropic hormone, arginine vasopressin, and corticotropin-releasing hormone; said metabolic biomarkers comprise one or more of adiponectin, acylation stimulating protein, fatty acid binding protein, insulin, leptin, prolactin, resistin, testosterone, and thyroid stimulating hormone; and said neurotrophic biomarkers comprise one or more of brain-derived neurotrophic factor, S100B, neurotrophin 3, glial cell line-derived neurotrophic factor, reelin and isoforms thereof, and artemin; (b) applying binary logistic regression to said measured levels to obtain a hypermap vector for each group of biomarkers; and (c) obtaining a hypermap that comprises vectors obtained for measured levels of said groups of biomarkers in biological samples from control subjects identified as having MDD, and vectors obtained for measured levels of said groups of biomarkers in biological samples from control subjects identified as not having MDD; (d) comparing the hypermap vectors for said individual to the hypermap to determine the probability that said individual has MDD.

The above method can further comprise, if it is determined in step (d) that said individual is likely to have MDD: (e) comparing hypermap vectors for said individual prior to and subsequent to therapy for said MDD to said hypermap to determine whether a change has occurred.

In one aspect, this document discloses a method for assessing the likelihood that an individual has MDD, comprising

(a) identifying groups of biomarkers that may be related to MDD;
(b) obtaining clinical data from a plurality of subjects for the identified groups of biomarkers, wherein some of the subjects are diagnosed as having MDD and some of the subjects do not have MDD;
(c) applying optimization algorithms to the clinical data and calculating coefficients for selected biomarkers within each group;
(d) creating a hypermap by generating vectors for each group of selected biomarkers;
(e) measuring the levels of said selected biomarkers in one or more biological samples from said subject;
(f) applying said algorithms to said measured levels; and
(g) comparing the result of said algorithms for said individual to the hypermap to determine whether said individual is likely to have MDD, is not likely to have MDD, or falls into a sub-class that can be used to predict disease course, select a treatment regimen, or provide information regarding severity. The method can be an *in vitro* method.

The method can further comprise, if it is determined in step (g) that said individual is likely to have MDD, comparing the result of hypermaps for said individual prior to and subsequent to therapy for said MDD, determining whether a change in biomarker pattern has occurred, and determining whether any such change is reflected in the clinical status of the individual.

The groups of biomarkers can include two or more inflammatory biomarkers, HPA axis biomarkers, metabolic biomarkers, or neurotrophic biomarkers. The inflammatory biomarkers can be selected from the group consisting of alpha 1 antitrypsin, alpha 2 macroglobin, apolipoprotein CIII, CD40 ligand, interleukin 6, interleukin 13, interleukin 18, interleukin 1 receptor antagonist, myeloperoxidase, plasminogen activator inhibitor-1, RANTES (CCL5), tumor necrosis factor alpha (TNF$\alpha$), sTNFRI, and sTNFRII. The HPA axis biomarkers can be selected from the group consisting of cortisol, epidermal growth factor, granulocyte colony stimulating factor, pancreatic polypeptide, adrenocorticotropic hormone, arginine vasopressin, and corticotropin-releasing hormone. The metabolic biomarkers can be selected from the group consisting of adiponectin, acylation stimulating protein, fatty acid binding protein, insulin, leptin, prolactin, resistin, testosterone, and thyroid stimulating hormone. The neurotrophic biomarkers can be selected from the group consisting of brain-derived neurotrophic factor, S100B, neurotrophin 3, glial cell line-derived neurotrophic factor, artemin, and reelin and its isoforms.

Unless otherwise defined, all technical and scientific terms used herein have the same meaning as commonly understood by one of ordinary skill in the art to which this invention pertains. In case of conflict, the present specification, including definitions, will control.

## BRIEF DESCRIPTION OF THE DRAWINGS

FIG. 1 is a diagram depicting steps that can be included in some embodiments of a method for generating a hypermap for particular disease.

FIG. 2 is a diagram depicting steps that can be included in some embodiments of a process for constructing a hypermap from selected groups of markers and clinical data for a particular disease.

FIG. 3 is a hypermap representation of patients diagnosed with MDD (asterisks) and a normal control group (circles).

FIG. 4 is a graph illustrating the results of applying a formula to a set of clinical samples from MDD patients (black bars) as compared to age-matched healthy normal subjects (gray bars). The test score represents 10 times the probability that a subject has MDD (10 x $P_{MDD}$).

FIG. 5 is a hypermap representation of clinical data from a longitudinal study of a group of drug naïve MDD patients whose sera were tested prior to and 2 and 8 weeks after initiation of therapy with the antidepressant LEXAPRO™. Vectors indicate the change in the biomarker pattern subsequent to treatment.

## DETAILED DESCRIPTION

MDD, also known as major depression, unipolar depression, clinical depression, or simply depression, is a mental disorder characterized by a pervasive low mood and loss of interest or pleasure in usual activities. A diagnosis of MDD typically is made if a person has suffered one or more major depressive episodes. MDD affects nearly 19 million Americans annually. The most common age of onset is between 30 and 40 years, with a later peak between 50 and 60 years of age. Diagnosis generally is based on a subject's shelf-reported experiences and observed behavior. Biobehavioral research, however, is among the most challenging of scientific endeavors, since biological organisms display wide-ranging individual differences in physiology. In particular, the paradigm used for neuropsychiatric diagnosis and patient management is based upon clinical interviews to stratify patients within adopted classifications. This paradigm has the caveat of not including information derived from biological or pathophysiological mechanisms. There remains a need for a reliable method to diagnose or determine predisposition to depression disorders, or to assess a subject's disease status and/or response to treatment. As described herein, biomarker hypermapping (BHM) technology represents a methodology to both visualize patterns associated with the disease state as well as sub-classification of patient groups or individual patients based upon a pattern.

Commonly, methods related to multi-analyte diagnostics typically use either a global optimization method in which all the markers (parameters) are used in multivariable optimization to best fit the clinical study results, or use a decision tree methodology. Decision trees can be used to determine the best way to distinguish individuals with a disease from normal subjects in a clinical setting. Many of these methods are effective when the number of analyzes are small (typically less than 5). In such situations, experts as well as those less skilled can make a diagnosis independent of significant insight into the underlying biology of the disease or the tests employed. For complex diseases, however, where symptoms overlap and there can be significant variation between stages of disease, a larger number of analytes are required to diagnose or sub-classify patients. In such cases, many parameters need to be taken into account, and the contribution of each parameter (analyte) is small. Even experts can have a hard time gaining insight into the status of an individual patient. Similarly, medical researchers looking at the underlying biology of a disease or hoping to develop new therapeutics may miss useful information by performing a simple global optimization.

The BMH approach uses biomarkers reflective of different physiologic parameters (e.g., hormones, metabolic markers, and inflammatory markers) to construct a visualization of changes in biomarker expression that may be related to disease state. In this process, a patient's biomarker responses are mapped onto a multi-dimensional hyperspace. Distinct coefficients can be derived to create hyperspace vectors for subsets of patients and age-matched normal subjects. Multiplex biomarker data from clinical sample sets can be used iteratively to construct and define a hyperspace map, which then can be used to separate disease states from normal states and provide guidance in treatment plans.

In general, the methods described herein are directed to analysis of multi-analyte diagnostic tests. These methods can be particular useful with complex diseases, for which it often is difficult to identify one or two markers that will provide enough unique separation between patient sub-groups, e.g., those with a different prognosis or manifestation of disease or, as often occurs with behavioral diseases, distinguishing affected from normal subjects. Multiple markers (e.g., 2, 3, 4, 5, or more than 5 markers) can be used in combination in the presently described methods to provide increased power of a diagnostic test, allowing clinicians to discriminate between patients and prevent confounding co-morbidities from other diseases from interfering with sensitivity and specificity, for example.

Different groups of markers can be selected based on physiologic/biologic functions related to a disease of interest by use of direct analysis of clinical studies and/or bioinformatics. Using a large library of biomarkers, markers can be grouped according to functional activity that reflects different segments of human physiology and/or biologic processes. Within each group, multiple markers can be used to provide an accurate measurement of the physiologic or biologic changes within each process or system. For analysis of complex diseases, multiple groups can be used for measurement of whole body changes under a particular disease condition.

Rather than performing a global optimization for all measured markers in all related groups within a body of clinical study data, the methods provided herein can first include optimization of the measured markers in each functional group using clinical study data. The optimized results for each group can be used to construct a combination parameter that represents the group in the construction of a preliminary hypermap of the disease. Data from multiple studies can be used iteratively to further develop the disease hypermap. The data from individual patients then can be mapped to the disease hypermap in order to take advantage of what is known about previously characterized patients whose biomarker profiles fall within the same multi-dimensional space. Knowledge gained from analysis of previously characterized patients can be used to sub-categorize the patient, predict disease course, and make decisions regarding, for example, treatment options (e.g., drugs of choice and other potentially successful therapeutic approaches).

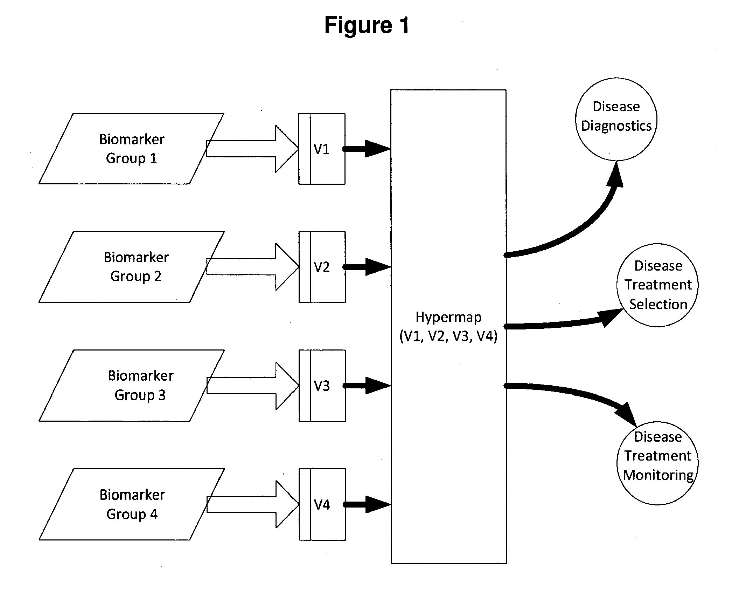

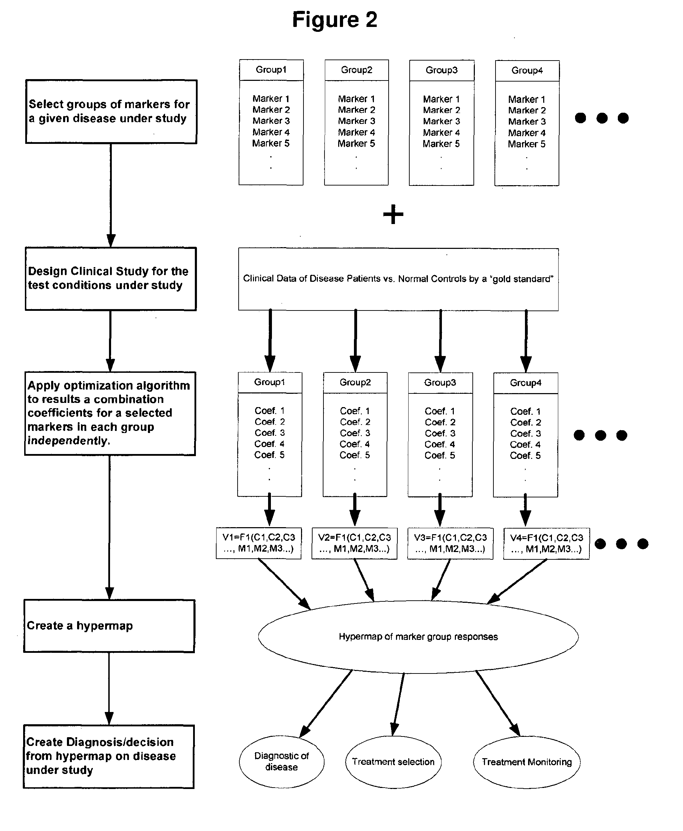

Figures 1 and 2 illustrate processes for constructing hypermaps from selected groups, markers, and clinical data for a given disease. As shown, several steps can be used to create a hypermap for a disease of interest. In some embodiments, the first step can be to select groups of markers, based on the physiology and biology of the disease, as well as current understanding of biomarker responses within the disease state. Many diseases have shared elements that include inflammation, tissue remodeling, metabolic changes, immune response, cell migration, hormonal imbalance, etc. Certain diseases are associated with pain or neurologic dysfunction, or there may be specific markers that are characteristic of a specific disease (e.g., elevated blood glucose in diabetes) or response to a specific drug (e.g., estrogen receptor expression in breast cancer patients). Biomarkers can be grouped differently, essentially via functional clustering, which can provide more information relative to the pathways involved in physiological dysfunctions. In inflammation, for example, markers can include those related to the acute phase response (e.g., C-reactive protein), the cytokine response (e.g., Th1- and Th2-related interleukins), chemokines, and chemoattractant molecules (e.g., IL-8 in the attraction of neurophils into the lung that is characteristic of certain respiratory diseases). The following paragraphs set forth exemplary groups of biomarkers.

*Inflammatory Biomarkers*

A large variety of proteins are involved in inflammation, and all are open to genetic mutations that can impair or otherwise dysregulate normal expression and function. Inflammation also induces high systemic levels of acute-phase proteins. These include C-reactive protein, serum amyloid A, serum amyloid P, vasopressin, and glucocorticoids, which can cause a range of systemic effects. In addition, proinflammatory cytokines and chemokines are involved in inflammation. Table 1 provides an exemplary list of inflammatory biomarkers.

**Table 1**

| Gene Symbol | Gene Name | Cluster |
|---|---|---|
| A1AT | Alpha 1 Antitrypsin | Inflammation |
| A2M | Alpha 2 Macroglobin | Inflammation |
| AGP | Alpha 1-Acid Glycoprotein | Inflammation |
| ApoC3 | Apolipoprotein CIII | Inflammation |
| CD40L | CD40 ligand | Inflammation |
| IL-1($\alpha$ or $\beta$) | Interleukin 1 | Inflammation |
| IL-6 | Interleukin 6 | Inflammation |
| IL-13 | Interleukin 13 | Inflammation |
| IL-18 | Interleukin 18 | Inflammation |
| IL-1ra | Interleukin 1 Receptor Antagonist | Inflammation |
| MPO | Mveloperoxidase | Inflammation |
| PAI-1 | Plasminogen activator inhibitor-1 | Inflammation |
| RANTES | RANTES (CCL5) | Inflammation |
| TNFA | Tumor Necrosis Factor alpha | Inflammation |
| STNFR | Soluble TNF$\alpha$receptor (I,II) | Inflammation |

*HPA Axis Biomarkers*

The hypothalamic-pituitary-adrenal axis (HPA or HTPA axis), also known as the limbic-hypothalamic-pituitary-adrenal axis (LHPA axis), is a complex set of direct influences and feedback interactions among the hypothalamus, the pituitary gland, and the adrenal (or suprarenal) glands. The interactions among these organs constitute the HPA axis, a major part of the neuroendocrine system that controls reactions to stress and regulates many body processes, including digestion, the immune system, mood and emotions, sexuality, and energy storage and expenditure. Examples of HPA biomarkers include ACTH and cortisol, as well as others listed in Table 2.

**Table 2**

| Gene Symbol | Gene Name | Cluster |
|---|---|---|
| None | Cortisol | HPA axis |
| EGF | Epidermal Growth Factor | HPA axis |
| GCSF | Granulocyte Colony Stimulating Factor | HPA axis |
| PPY | Pancreatic Polypeptide | HPA axis |
| ACTH | Adrenocorticotropic hormone | HPA axis |
| AVP | Arginine Vasopressin | HPA axis |
| CRH | Corticotropin-Releasing Hormone | HPA axis |

*Metabolic biomarkers*

Metabolic biomarkers provide insight into metabolic processes in wellness and disease states. Human diseases manifest in complex downstream effects, affecting multiple biochemical pathways. Proteins and hormones controlling these processes, as well as metabolites can be used for diagnosis and patient monitoring. Table 3 provides an example of a list of metabolic biomarkers that can be assessed using the methods described herein.

**Table 3**

| Gene Symbol | Gene Name | Cluster |
|---|---|---|
| ACRP30 | Adiponectin | Metabolic |
| ASP | Acylation Stimulating Protein | Metabolic |
| FABP | Fatty Acid Binding Protein | Metabolic |
| INS | Insulin | Metabolic |
| LEP | Leptin | Metabolic |
| PRL | Prolactin | Metabolic |
| RETN | Resistin | Metabolic |
| None | Testosterone | Metabolic |
| TSH | Thyroid Stimulating Hormone | Metabolic |
| None | Thyroxine | Metabolic |

*Neurotrophic factors*

Neurotrophic factors are a family of proteins that are responsible for the growth and survival of developing neurons and the maintenance of mature neurons. Neurotrophic factors have been shown to promote the initial growth and development of neurons in the central nervous system (CNS) and peripheral nervous system (PNS), and to stimulate regrowth of damaged neurons in test tubes and animal models. Neurotrophic factors often are released by the target tissue in order to guide the growth of developing axons. Most neurotrophic factors belong to one of three families: (1) neurotrophins, (2) glial cell-line derived neurotrophic factor family ligands (GFLs), and (3) neuropoietic cytokines. Each family has its own distinct signaling pathway, although the cellular responses that are elicited often overlap. An exemplary list of neurotrophic biomarkers is presented in Table 4. Reelin is a protein that helps regulate processes of neuronal migration and positioning in the developing brain. Besides this important role in early development, reelin continues to work in the adult brain by modulating synaptic plasticity by enhancing the induction and maintenance of long-term potentiation. Reelin has been implicated in the pathogenesis of several brain diseases. Significantly lowered expression of the protein has been observed in schizophrenia and psychotic bipolar disorder. Serum levels of certain reelin isoforms may differ in MDD and other mood disorders, such that measurement of reelin isoforms can enhance the ability to distinguish MDD from bipolar disease and schizophrenia, as well as further sub-classify patient populations.

**Table 4**

| Gene Symbol | Gene Name | Cluster |
|---|---|---|
| BDNF | Brain-derived neurotrophic factor | Neurotrophic |
| S100B | S100B | Neurotrophic |
| NTF3 | Neurotrophin 3 | Neurotrophic |
| RELN | Reelin | Neurotrophic |
| GDNF | Glial cell line derived neurotrophic factor | Neurotrophic |
| ARTN | Artemin | Neurotrophic |

*Methods for Using Hypermapping Information*

Information regarding biomarkers and hypermapping as discussed herein can be used for, without limitation, treatment monitoring. For example, hypermapping information can be provided to a clinician for use in establishing or altering a course of treatment for a subject. When a treatment is selected and treatment starts, the subject can be monitored periodically by collecting biological samples at two or more intervals, generating hypermapping information corresponding to a given time interval pre- and post-treatment, and comparing the result of hypermaps over time. On the basis of such hypermapping information and any trends observed with respect to increasing, decreasing, or stabilizing biomarker levels, for example, a clinician, therapist, or other health-care professional may choose to continue treatment as is, to discontinue treatment, or to adjust the treatment plan with the goal of seeing improvement over time.

After a patient's biomarker and/or hypemapping information is reported, a health-care professional can take one or more actions that can affect patient care. For example, a health-care professional can record the information and biomarker expression levels in a patient's medical record. In some cases, a health-care professional can record a diagnosis of a neuropsychiatric disease, or otherwise transform the patient's medical record, to reflect the patient's medical condition. In some cases, a health-care professional can review and evaluate a patient's medical record, and can assess multiple treatment strategies for clinical intervention of a patient's condition.

For major depressive disorder and other mood disorders, treatment monitoring can help a clinician adjust treatment dose(s) and duration. An indication of a subset of alterations in hypermapping information that more closely resemble normal homeostasis can assist a clinician in assessing the efficacy of a regimen. A health-care professional can initiate or modify treatment for symptoms of depression and other neuropsychiatric diseases after receiving information regarding a patient's hypermapping result. In some cases, previous reports of hypermapping information can be compared with recently communicated hypermapping information. On the basis of such comparison, a health-care profession may recommend a change in therapy. In some cases, a health-care professional can enroll a patient in a clinical trial for novel therapeutic intervention of MDD symptoms. In some cases, a health-care professional can elect waiting to begin therapy until the patient's symptoms require clinical intervention.

A health-care professional can communicate information regarding or derived from hypermapping to a patient or a patient's family. In some cases, a health-care professional can provide a patient and/or a patient's family with information regarding MDD, including treatment options, prognosis, and referrals to specialists, e.g., neurologists and/or counselors. In some cases, a health-care professional can provide a copy of a patient's medical records to communicate hypermapping information to a specialist.

A research professional can apply information regarding a subject's hypermapping information to advance MDD research. For example, a researcher can compile data on hypermaps with information regarding the efficacy of a drug for treatment of depression symptoms, or the symptoms of other neuropsychiatric diseases, to identify an effective treatment. In some cases, a research professional can obtain a subject's hypermapping information to evaluate a subject's enrollment or continued participation in a research study or clinical trial. In some cases, a research professional can communicate a subject's hypermapping information to a health-care professional, and/or can refer a subject to a health-care professional for clinical assessment and treatment of neuropsychiatric disease.

Any appropriate method can be used to communicate information to another person (e.g., a professional), and information can be communicated directly or indirectly. For example, a laboratory technician can input vector information, biomarker levels, and/or hypermapping outcome information into a computer-based record. In some cases, information can be communicated by making a physical alteration to medical or research records. For example, a medical professional can make a permanent notation or flag a medical record for communicating a diagnosis to other health-care professionals reviewing the record. Any type of communication can be used (e.g., mail, e-mail, telephone, facsimile and face-to-face interactions). Secure types of communication (e.g., facsimile, mail, and face-to-face interactions) can be particularly useful. Information also can be communicated to a professional by making that information electronically available (e.g., in a secure manner) to the professional. For example, information can be placed on a computer database such that a health-care professional can access the information. In addition, information can be communicated to a hospital, clinic, or research facility serving as an agent for the professional. Information transferred over open networks (e.g., the internet or e-mail) can be encrypted. When closed systems or networks are used, existing access controls may be sufficient.

Examples not pertaining to the invention are for illustrative purposes only.

## EXAMPLES

Example 1 - Biological Hypermapping for MDD

To populate each group of biomarkers for a particular clinical condition, a list of marker candidates is selected that best reflects the state of the group reflective to changes in the condition. In the case of MDD, candidate biomarkers were selected based upon clinical studies, and were sub-classified using a bioinformatic approach based on their role in MDD. The biomarkers utilized in the present example are listed in Tables 1 to 3 above.

While any combination of the markers in each group could have been used to construct a hyperspace vector ($V_1...V_n$), the biomarkers that were used were taken from a library of biomarker tests that previously had been evaluated for their suitability for quantitative measurement, based on the accuracy and precision of the assay in biological fluids (particularly blood, serum, and plasma).

The second step in the processes provided herein typically is to design and collect clinical study data. Clinical samples are collected from patients having the disease of interest. Samples are collected from patients that typically have been diagnosed by known "gold standard" criteria. A set of age- and gender-matched samples also is obtained from normal subjects. The patient samples can be from a group of subjects with different disease states/severities/treatment choices/treatment outcomes, for example. Patient selection criteria depend upon the test outcome understudied. In the case of MDD, patients with different disease severities, durations, reoccurrences, treatment options (e.g., different classes of antidepressants), and treatment outcomes were selected. Normal subjects were required to have no history of depression, both personally and in their immediate family members, in addition to being free form confounding diseases.

The third step of the methods provided herein typically is to use the measured marker data from the clinical study samples to construct a hyperspace vector from each group of markers. There are several choices of algorithms for constructing hyperspace vectors. The chosen method generally depends on the disease conditions under study. For example, in the development of a diagnostic test for MDD, the clinical result is depressed vs. not depressed. Thus, a binary logistic regression optimization is used to fit the clinical data with selected markers in each group against the clinical results from "gold standard" diagnosis. The result of the fit is a set of coefficients for the list of markers in the group. For example, A1AT (I1), A2M (I2), apolipoprotein CIII (I3), and TNF alpha (I4) were selected as the four markers representing the inflammatory group. Using binary logic regression against clinical results, four coefficients and the constants for these markers were calculated. The vector for the inflammatory group was constructed as follows:

$$V_{infla} = 1/(1+ \exp\text{-}(CI0 + CI1*I1 + CI2*I2+CI3*I3+CI4*I4)) \qquad (1)$$

Where CI0 = -7.34
CI1= -0.929
CI2=1.10
CI3=5.13
CI4=6.48

$V_{infla}$ represented the probability of whether a given patient had MDD using the measured inflammatory markers.

In the same way, vectors for other groups of markers were derived for MDD. Four markers were chosen to represent the metabolic group: M1=ASP, M2=prolactin, M3=resistin, and M4=testosterone. Using the same method of binary logistic regression described above for the clinical data, a set of coefficients and a vector summary were developed for patient metabolic response:

$$V_{meta} = 1/(1+ \exp \text{-}(Cm0+Cm1*M1+Cm2*M2+Cm3*M3+Cm4*M4)) \qquad (2)$$

Where Cm0 = -1.10
Cm1=0.313
Cm2=2.66

Cm3=0.82
Cm4=-1.87

$V_{meta}$ represented the probability of whether a given patient had MDD using the measured metabolic markers.

Two markers were chosen to represent the HPA group: H1=EGF and H2=G-CSF. Again, using the same method of binary logistic regression on the clinical data as above, a set of coefficients and a vector summary were developed for patient HPA response:

$$V_{hpa} = 1/(1+ \exp -(Ch0+Ch1*H1+Ch2*H2)) \qquad (3)$$

Where Ch0 = -1.87
Ch1=7.33
Ch2=0.53

$V_{hpa}$ represented the probability of whether a given patient has MDD using the measured HPA markers.

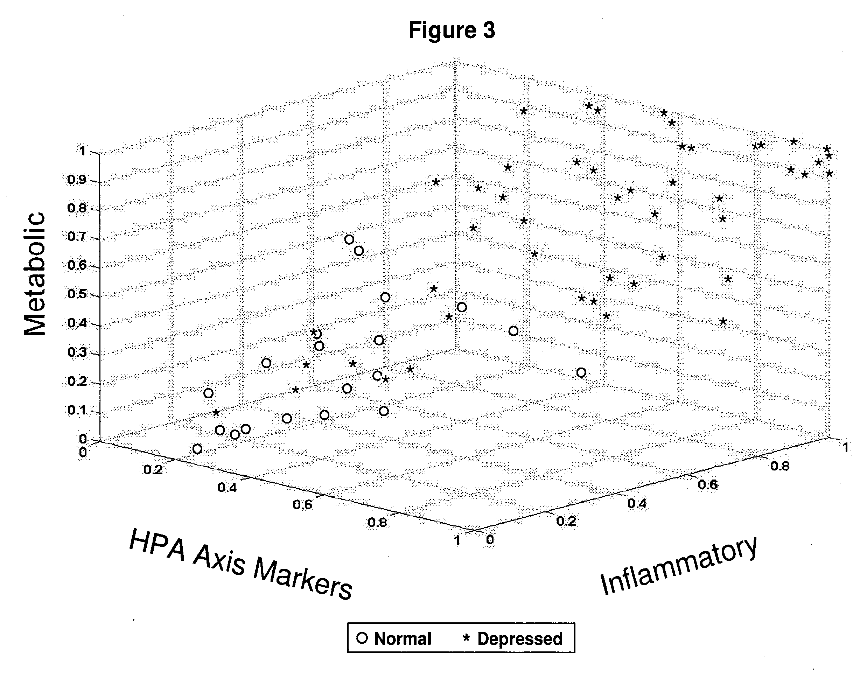

Using these three parameters, a hypermap for MDD was constructed. Figure 3 is a hypermap representation of patients diagnosed with MDD and a normal subject control group. This hypermap was constructed using data collected from the subjects by measurement and analysis of inflammatory, metabolic, and HPA marker groups. Asterisks represent patients with MDD, while circles represent normal subjects.

The last step of the methods described herein typically is to construct a diagnostic based on the hypermap. When correct marker groups and markers are selected, a hypermap for the disease can be constructed so that disease patients and healthy controls are represented in different regions of the hypermap. One can use a hypermap for simple one parameter diagnostics (e.g., the likelihood that an individual has a disease). Alternatively, one can construct more complicated diagnostics, perhaps indicating whether a particular patient will react with particular treatments, depending on the region of the hypermap into which the patient's marker response set falls. Such methods also can be used to determine whether a patient or falls into a specific sub-class that can be used to predict disease course, select a specific treatment regimen, or provide information regarding disease severity, for example.

In some cases, a method as provided herein can further include, if it is determined that a patient is likely to have MDD, comparing the result of hypermaps for the patient prior to and subsequent to therapy for the MDD, determining whether a change in biomarker pattern has occurred, and determining whether any such change is reflected in the clinical status of the patient. Accumulation of sufficient data on individual patients would allow for prediction of certain aspects of response to a specific treatment (e.g., an antidepressant, psychotherapy, or cognitive behavior modification), such as a positive or negative response or a profile for a specific side effect (e.g., sexual dysfunction or loss of libido).

To generate patient specific data, blood was drawn, the concentrations of selected markers in the plasma or sera were measured, and the measured marker concentration data were added into the formula, resulting in a diagnostic test score for MDD specific to individual patients. This method is also useful for optimizing treatment, for example. By hypermapping patients to a master hypermap derived from a large number of patients from whom clinical data is available, including data with regard to response to specific drugs, the response to a specific drug can be estimated based on the response of MDD patients with similar characteristics.

In the present example, a simple diagnostic for MDD was developed by combining three hypermap vectors ($V_{infa}$, $V_{HPA}$, and $V_{Meta}$) using a binary logic regression against clinical data to build a formula for the likelihood of patient having MDD. This resulted in equation (4):

$$P_{MDD} = 1/(1+Exp - (Cp0 + Cp1*V_{infla} + Cp2*V_{meta} + Cp3*V_{hpa})) \qquad (4)$$

Where Cp0 = -3.87
Cp1=5.46
Cp2=3.47
Cp3=-0.66

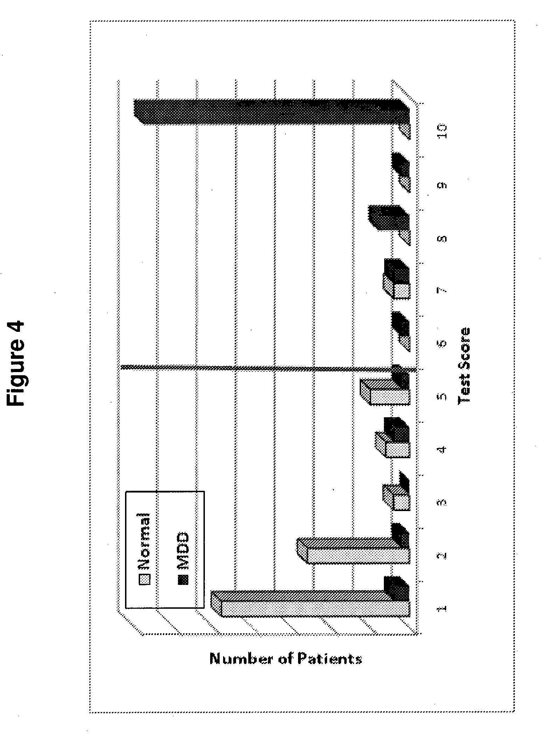

$P_{MDD}$ represents the probability of whether a patient has MDD using groups of markers from the inflammatory, metabolic, and HPA groups. Figure 4 illustrates the results of applying the formula to a set of clinical samples from MDD patients and age-matched control subjects. The test score = 10 x $P_{MDD}$.

The same method is used with different markers in the different groups to construct a hypermap, which in turn can be used to construct diagnostic tests. For example, one or more markers in the inflammatory, metabolic, and/or HPA groups are replaced to construct a hypermap and generate a diagnostic. Alternatively or in addition, neurotrophic marker groups are included to construct a mood disorder (e.g., MDD or bipolar disease) hypermap and generate a diagnostic formula. In the present example, where the question to be tested was whether or not a subject had MDD, binary logistic regression was used to construct hypermap group vectors. It is noted that other regression methods also can be used to construct the vectors for more complicated questions and/or situations.

Example 2 - Use of hypermapping to assess changes in disease state

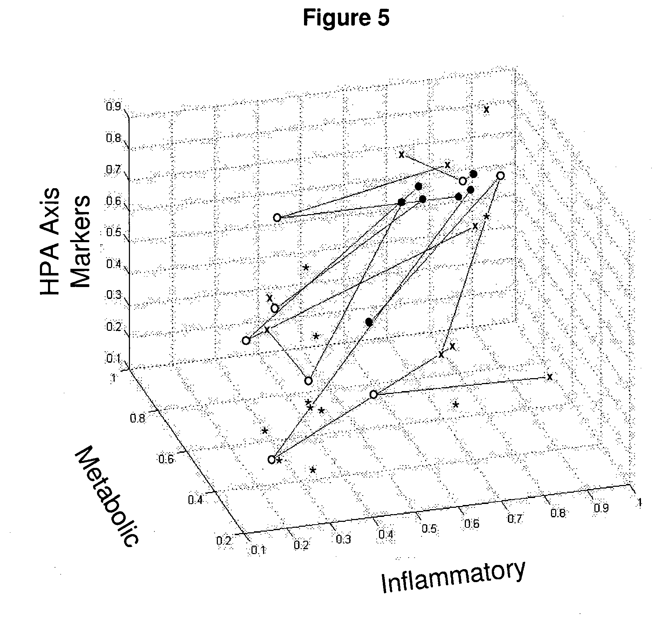

As noted above, certain external factors, diseases, and therapeutics can influence the expression of one or more biomarkers that are components of a vector within a hypermap. Figure 5 is a hypermap that was developed to demonstrate the response pattern for a series of MDD patients who initiated therapy with the antidepressant LEXAPRO™. Figure 5 shows changes in BHYPERMAP™ in a subset of Korean MDD patients after treatment with LEXAPRO™. MDD patients at baseline are red dots. After 2 -3 weeks of treatment the dots are yellow, and after 8 weeks of treatment the dots are blue. The green dots represent normal subjects. This demonstrates that the technology described herein can be used to define changes in an individual pattern in response to antidepressant therapy.

**Claims**

1. An *in vitro* method for assessing the probability that an individual has major depressive disorder (MDD), comprising

   (a) measuring the levels of two or more groups of biomarkers in one or more serum, plasma, or blood samples from said subject, wherein said two or more groups of biomarkers are relevant to MDD and are selected from the group consisting of inflammatory biomarkers, hypothalamic-pituitary-adrenal (HPA) axis biomarkers, metabolic biomarkers, and neurotrophic biomarkers, wherein each group comprises two or more biomarkers; and wherein
   said inflammatory biomarkers comprise one or more of alpha 1 antitrypsin, alpha 2 macroglobulin, apolipoprotein CIII, CD40 ligand, interleukin 6, interleukin 13, interleukin 18, interleukin 1 receptor antagonist, myeloperoxidase, plasminogen activator inhibitor-1, RANTES (CCL5), soluble tumor necrosis factor alpha receptor II, and tumor necrosis factor alpha;
   said HPA axis biomarkers comprise one or more of cortisol, epidermal growth factor, granulocyte colony stimulating factor, pancreatic polypeptide, adrenocorticotropic hormone, arginine vasopressin, and corticotropin-releasing hormone;
   said metabolic biomarkers comprise one or more of adiponectin, acylation stimulating protein, fatty acid binding protein, insulin, leptin, prolactin, resistin, testosterone, and thyroid stimulating hormone; and
   said neurotrophic biomarkers comprise one or more of brain-derived neurotrophic factor, S100B, neurotrophin 3, glial cell line-derived neurotrophic factor, reelin and isoforms thereof, and artemin;
   (b) applying binary logistic regression to said measured levels to obtain a hypermap vector for each group of biomarkers; and
   (c) obtaining a hypermap that comprises vectors obtained for measured levels of said groups of biomarkers in biological samples from control subjects identified as having MDD, and vectors obtained for measured levels of said groups of biomarkers in biological samples from control subjects identified as not having MDD;
   (d) comparing the hypermap vectors for said individual to the hypermap of step (c) to determine the probability that said individual has MDD.

2. The method of claim 1, further comprising, if it is determined in step (d) that said individual is likely to have MDD:

   (e) comparing hypermap vectors for said individual prior to and subsequent to therapy for said MDD to said hypermap to determine whether a change has occurred.

**Patentansprüche**

1. Ein *in vitro* Verfahren zur Bemessung der Wahrscheinlichkeit, dass ein Individuum eine schwere depressive Erkrankung (MDD) hat, umfassend:

   (a) Messung der Spiegel von zwei oder mehreren Gruppen von Biomarkern in einer oder mehreren Serum-, Plasma-, oder Blutprobe(n) von jenem Subjekt, wobei jene zwei oder mehr Gruppen von Biomarkern relevant für MDD sind und ausgewählt sind aus der Gruppe bestehend aus inflammatorischen Biomarkern, Hypothalamus-Hypophysen-Nebennierenrinden (HPA)-Achse-Biomarkern, metabolischen Biomarkern und neurotrophen Biomarkern, wobei jede Gruppe zwei oder mehr Biomarker umfasst; und wobei

jene inflammatorischen Biomarker einen oder mehrere aus alpha-1-Antitrypsin, alpha-2-Makroglobulin, Apolipoprotein CIII, CD40-Ligand, Interleukin-6, Interleukin-13, Interleukin-18, Interleukin-1-Rezeptorantagonist, Myeloperoxidase, Plasminogen-Aktivator-Inhibitor-1, RANTES (CCL5), löslicher Tumornekrosefaktor-alpha-Rezeptor-II und Tumornekrosefaktor-alpha umfassen;

jene HPA-Achse-Biomarker einen oder mehrere aus Cortisol, epidermaler Wachstumsfaktor, Granulozyten-Kolonie stimulierender Faktor, pankreatisches Polypeptid, adrenocorticotropes Hormon, Arginin-Vasopressin und Corticotropin-freisetzendes Hormon umfassen;

jene metabolischen Biomarker einen oder mehrere aus Adiponektin, Acylierung-stimulierendes Protein, Fettsäure-bindendes Protein, Insulin, Leptin, Prolaktin, Resistin, Testosteron und Thyreoidea-stimulierendes Hormon umfasst; und

jene neurotrophen Biomarker einen oder mehrere aus "Brain-derived-neurotrophic-Factor", S100B, Neurotrophin-3, Gliazelllinie-gewonnener neurotropher Faktor, Reelin und Isoformen davon, und Artemin umfasst;

(b) Anwendung binärer logistischer Regression auf jene gemessenen Spiegel um einen Hypermap-Vektor für jede Gruppe von Biomarkern zu erhalten; und

(c) Erlangung einer Hypermap, die Vektoren umfasst erhalten für gemessene Spiegel jener Gruppen von Biomarkern in biologischen Proben von Kontroll-Subjekten , die identifiziert wurden MDD zu haben, und Vektoren erhalten für gemessene Spiegel jener Gruppen von Biomarkern in biologischen Proben von Kontroll-Subjekten, die identifiziert wurden nicht MDD zu haben;

(d) Vergleich der Hypermap-Vektoren für jenes Individuum mit der Hypermap aus Schritt (c) um die Wahrscheinlichkeit, dass jenes Individuum MDD hat, zu bestimmen.

2. Das Verfahren aus Anspruch 1, ferner umfassend, falls in Schritt (d) festgestellt wurde, dass jenes Individuum wahrscheinlich MDD hat:

(e) Vergleichen von Hypermap-Vektoren für jenes Individuum bevor und im Anschluss an die Therapie für jene MDD mit jener Hypermap, um festzustellen, ob eine Veränderung eingetreten ist.

## Revendications

1. Procédé *in vitro* permettant d'évaluer la probabilité qu'un individu souffre d'un trouble dépressif majeur (TDM), comprenant

(a) la mesure des taux de deux groupes ou plus de biomarqueurs dans un ou plusieurs échantillons de sérum, de plasma ou de sang provenant dudit sujet, dans lequel lesdits deux groupes ou plus de biomarqueurs sont pertinents avec le TDM et sont choisis dans le groupe constitué des biomarqueurs inflammatoires, des biomarqueurs de l'axe hypothalamo-hypophyso-surrénalien (HHS), des biomarqueurs métaboliques et des biomarqueurs neurotrophiques, dans lequel chaque groupe comprend deux biomarqueurs ou plus ; et dans lequel lesdits biomarqueurs inflammatoires comprennent un ou plusieurs parmi l'alpha-1-antitrypsine, l'alpha-2-macroglobuline, l'apolipoprotéine CIII, le ligand du CD40, l'interleukine 6, l'interleukine 13, l'interleukine 18, un antagoniste du récepteur de l'interleukine 1, la myéloperoxydase, l'inhibiteur 1 de l'activateur du plasminogène, RANTES (CCL5), le récepteur II du facteur de nécrose tumorale alpha soluble, et le facteur de nécrose tumorale alpha ;

lesdits biomarqueurs de l'axe HHS comprennent un ou plusieurs parmi le cortisol, le facteur de croissance des cellules épidermiques, le facteur de stimulation des colonies de granulocytes, le polypeptide pancréatique, l'hormone adrénocorticotrope, l'arginine-vasopressine, et l'hormone de libération de la corticotropine ;

lesdits biomarqueurs métaboliques comprennent un ou plusieurs parmi l'adiponectine, la protéine de stimulation de l'acylation, la protéine de liaison des acides gras, l'insuline, la leptine, la prolactine, la résistine, la testostérone, et l'hormone de stimulation de la thyroïde ; et

lesdits biomarqueurs neurotrophiques comprennent un ou plusieurs parmi le facteur neurotrophique dérivé du cerveau, S100B, la neurotrophine 3, le facteur neurotrophique dérivé de la lignée des cellules gliales, la Reelin et ses isoformes, et l'artémine ;

(b) l'application d'une régression logistique binaire auxdits taux mesurés pour obtenir un vecteur d'hyper-carte pour chaque groupe de biomarqueurs ; et

(c) l'obtention d'une hyper-carte qui comprend des vecteurs obtenus pour des taux mesurés desdits groupes de biomarqueurs dans des échantillons biologiques provenant de sujets témoins identifiés comme souffrant de TDM, et des vecteurs obtenus pour des taux mesurés desdits groupes de biomarqueurs dans des échantillons biologiques provenant de sujets témoins identifiés comme ne souffrant pas de TDM ;

(d) la comparaison des vecteurs d'hyper-carte pour ledit individu à l'hyper-carte de l'étape (c) pour déterminer la probabilité que ledit individu souffre de TDM.

2. Procédé selon la revendication 1, comprenant en outre, s'il est déterminé dans l'étape (d) que ledit individu souffre probablement de TDM :

(e) la comparaison des vecteurs d'hyper-carte pour ledit individu avant et après un traitement pour ledit TDM à ladite hyper-carte pour déterminer si un changement est survenu.

# Figure 1

EP 2 337 866 B1

# Figure 2

| Group1 | Group2 | Group3 | Group4 |
|---|---|---|---|
| Marker 1 | Marker 1 | Marker 1 | Marker 1 |
| Marker 2 | Marker 2 | Marker 2 | Marker 2 |
| Marker 3 | Marker 3 | Marker 3 | Marker 3 |
| Marker 4 | Marker 4 | Marker 4 | Marker 4 |
| Marker 5 | Marker 5 | Marker 5 | Marker 5 |
| . | . | . | . |

● ● ●

**Select groups of markers for a given disease under study**

**+**

**Design Clinical Study for the test conditions under study**

Clinical Data of Disease Patients vs. Normal Controls by a "gold standard"

**Apply optimization algorithm to results a combination coefficients for a selected markers in each group independently.**

| Group1 | Group2 | Group3 | Group4 |
|---|---|---|---|
| Coef. 1 | Coef. 1 | Coef. 1 | Coef. 1 |
| Coef. 2 | Coef. 2 | Coef. 2 | Coef. 2 |
| Coef. 3 | Coef. 3 | Coef. 3 | Coef. 3 |
| Coef. 4 | Coef. 4 | Coef. 4 | Coef. 4 |
| Coef. 5 | Coef. 5 | Coef. 5 | Coef. 5 |
| . | . | . | . |

● ● ●

$V1=F1(C1,C2,C3 \ldots, M1,M2,M3\ldots)$   $V2=F1(C1,C2,C3 \ldots, M1,M2,M3\ldots)$   $V3=F1(C1,C2,C3 \ldots, M1,M2,M3\ldots)$   $V4=F1(C1,C2,C3 \ldots, M1,M2,M3\ldots)$   ● ● ●

**Create a hypermap**

Hypermap of marker group responses

**Create Diagnosis/decision from hypermap on disease under study**

Diagnostic of disease

Treatment selection

Treatment Monitoring

Figure 3

Figure 4

**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MURRAY ; LOPEZ.** Global Health Statistics: A Compendium of Incidence, Prevalence and Mortality Estimates for over 2000 Conditions. Harvard School of Public Health, 1996
- **LÓPEZ-LEÓN et al.** *Molecular Psychiatry,* 2007, vol. 13 (8), 772-785
- **BERTHOLD-LOSLEBEN ; HIMMERICH.** *Current Neuropharmacology,* 2002, vol. 6 (3), 193-202
- **PARIANTE ; LIGHTMAN.** *Trends in Neurosciences,* 2008, vol. 31 (9), 464-468
- **HUNG et al.** *Clinical Endocrinology,* 2007, vol. 67 (5), 784-789
- **SCHROETER et al.** *Journal of Affective Disorders,* 2008, vol. 111 (2-3), 271-280